# EUROPEAN PATENT APPLICATION

(11) **EP 2 305 377 A1**
(43) Date of publication of application: **06.04.2011**
(21) Application number: 09012317.5
(22) Date of filing: 29.09.2009
(51) Int. Cl.: B01J 23/00, B01J 23/28, B01J 37/02

(54) **Catalysts for the preparation of cyanopyridines and their use**

(71) Applicant: Lonza Ltd., 4052 Basel (CH); POLYNT S.P.A., 24020 Scanzorosciate, BG (IT)
(72) Inventor: Zenklusen, Anton, 3937 Baltschieder (CH); Pianzola, Daniel, 3902 Brig-Glis (CH); Leanza, Roberto, 24040 Bergamo (IT); Mazzoni, Gianluca, 24020 Torre Boldone (IT); Armbruster, Erich, 3904 Naters (CH); Chuck, Roderick, 3902 Brig-Glis (CH)

(57) **Abstract**

The invention relates to methods for the production of cyanopyridines from alkylpyridines. The invention further relates to catalysts, uses of catalysts and methods for their production.

## Description

The invention relates to methods for the production of cyanopyridines from alkylpyridines. The invention further relates to catalysts, uses of catalysts and methods for their production.

### Background of the invention

Cyanopyridines are important starting materials for the production of pharmaceutical intermediates and other compounds. 3-Methylpyridine
(3-picoline) is an intermediate in the industrial production of nicotinic amide and nicotinic acid, which is an essential vitamin of the vitamin
B-complex (vitamin B₃).

Methods for the production of cyanopyridines from methylpyridines are known in the art. Commonly, the cyanopyridines are oxidized in the presence of a catalyst with ammonia and oxygen. The process is referred to as "ammoxidation" or "oxidative ammonolysis". Various catalysts are known, which comprise specific combinations of catalytic components, which can be coated on carrier materials.

Catalysts function to increase the rate of a chemical reaction at a given temperature by lowering the necessary amount of energy to reach the transition state. They can be present in the same phase as the reaction educts (homogenous catalysts) or in a different phase (heterogeneous catalysts).

WO 03/022819 discloses methods for the production of heteroaromatic nitriles by ammoxidation of the corresponding alkyl-substituted pyridines. The reaction is carried out with ammonia and an oxygen source in the presence of a catalyst based on oxides of vanadium and antimony and a promoter, such as chromium. The catalytic components are coated on a support material.

A catalyst based on vanadium, antimony and phosphorous is also disclosed in WO 2005/016505.

WO 2004/071657 discloses catalysts based on antimony and vanadium oxides for the production of nitriles by ammoxidation. Specifically, a catalyst is prepared with graphite and a silicate mineral.

Methods and catalysts for oxidative ammonolysis of alkylpyridines are also disclosed in WO 95/32054. The catalysts are based on vanadium, titanium and zirconium oxide, wherein titanium is induced in a molecular excess. Further components are co-precipitated in the production process and induced into the catalyst without a carrier material.

The production of cyanopyridines from alkylpyridines is an important industrial process. There is thus an ongoing need for providing alternative and improved catalysts. Specifically, there is a need for catalysts, which are easy to produce and highly efficient. Further, there is a need for catalysts which have a long lifetime, which remain stable for a long time and which can be manufactured from a limited number of components with low deviations in catalytic properties. The production of cyanopyridines from alkylpyridines is frequently not possible at the desired efficiency and continuity.

### Problem underlying the invention

The problem underlying the invention is to provide a method for the preparation of cyanopyridines and catalysts useful therefore, which overcome the above-mentioned disadvantages. Specifically, it is an object of the invention to provide a highly efficient catalyst, which is relatively easily available. Preferably, the catalyst shall be easy to manufacture, comprise only a low number of components and shall be available with low deviations in catalytic properties. The catalyst should be applicable in a standard industrial process for the production of cyanopyridines. The catalyst should have a long lifetime, whilst being stable against environmental influences, such as catalyst poisoning.

### Disclosure of the invention

Surprisingly, the problem underlying the invention is solved by catalysts and methods according to the claims.

Subject of the invention is a catalyst for the production of cyanopyridines from alkylpyridines, the catalyst comprising a coating comprising more than 50 weight % vanadium oxide and more than 2 weight % titanium oxide coated on a carrier, wherein the coating does not comprise antimony oxide. According to the invention, the vanadium oxide is preferably vanadium pentoxide (V₂O₅) and the titanium oxide is preferably titanium dioxide (TiO₂)**.** However, the compounds may comprise a certain amount of vanadium or titanium in a different oxidative state. For example, if the catalyst comprises vanadium pentoxide, up to 1 weight %, up to 2 weight % or up to 5 weight % V₂O₄, based on the total amount of vanadium oxides, may be present. Preferably, the amount of vanadium pentoxide, based on the total amount of vanadium oxides, is at least 95, 98 or 99 weight %. In a preferred embodiment of the invention, the coating further comprises molybdenum oxide, which preferably is MoO₃.

In the coating of the inventive catalyst, vanadium oxide and titanium oxide are the main components (in weight %). Specifically, vanadium oxide is the main component in the coating (in weight %). Preferably, the catalyst coating comprises more vanadium oxide than titanium oxide. Preferably, the coating comprises more than 60 or more than 70 weight % vanadium oxide. Preferably, the coating comprises between 3 and 40 weight %, preferably between 5 and 30 weight % titanium oxide.

In a preferred embodiment of the invention, the catalyst comprises or consists of
(a) 70 to 96. weight %, preferably 80 to 95 weight %, carrier
(b) 3 to 25 weight %, preferably 5 to 20 weight %, vanadium oxide
(c) 0.2 to 10 weight %, preferably 0.5 to 5 weight %, titanium oxide
(d) 0 to 5 weight %, preferably 0.1 to 2.5 weight %, molybdenum oxide.

The catalyst of the invention comprises a carrier and a coating. According to the present invention, "coating" refers to a solid surface cover, which is preferably applied to the carrier in a coating process. In a coating process, the coating is applied from a liquid solution, which provides a relatively even distribution of the coating on the carrier surface. In a preferred embodiment, the outer surface of the particles is fully coated. If the carrier is a porous material, the inner surface of the pores may be coated fully or at least partially. The skilled person knows that it depends on the pore size of porous materials and the conditions of the coating process, such as the viscosity of the coating solution, to what extent the pores are covered with the coating. In a preferred embodiment of the invention, the carrier is porous, and the pores are at least partially coated. In this embodiment, the pores preferably are of sufficient size that during the catalytic conversion of alkylpyridine, the gaseous phase passes the pores and thus the catalyst surface is increased by the pores.

According to the invention, the coating of the inventive catalyst comprises vanadium oxide, titanium oxide and preferably molybdenum oxide. In a preferred embodiment of the invention, the catalyst coating essentially consists of vanadium oxide and titanium oxide, or essentially consists of vanadium oxide, titanium oxide and molybdenum oxide. In this respect, "essentially" means that only these heavy metal coating components are included in the coating solution. However, the skilled person knows that the starting materials, such as metal oxides, may comprise impurities. Therefore, the catalyst coating of the present invention may comprise up to 0.05 weight%, up to 0.1 weight % or up to 0.5 weight % of additional metal oxides. Although an antimony source is not included in the coating process, the impurities may comprise low amounts of antimony. Further, the catalyst may comprise up to 5 weight %, up to 10 weight % or up to 20 weight % of other components, provided that no antimony oxide is included.

Surprisingly, it was found that a certain amount of molybdenum oxide enhances the catalytic efficiency. Preferably, in the catalyst of the invention, at least 0.1 weight % or at least 0.2 weight % of molybdenum oxide are included. Preferably, up to 1 weight %, up to 2 weight % or up to 5 weight % molybdenum oxide are included. In a preferred embodiment, a vanadium oxide is used as a starting compound which comprises molybdenum oxide as an impurity. This is advantageous, because impure vanadium oxide comprising a certain amount of molybdenum oxide is relatively inexpensive.

In another preferred embodiment of the invention, the molybdenum source (oxide) is added in pure form and distinct from the vanadium source.

In the inventive catalyst, the catalytic components are coated on a solid carrier (support). Preferably, the catalyst consists of the coating and the carrier. Preferably, the catalyst comprises at least 70, 80 or 85 weight % of the carrier.

In a preferred embodiment of the invention, the carrier comprises or consists of aluminium oxide and/or silicon oxide. In a preferred embodiment of the invention, the carrier comprises or consists of 5 to 40 weight % silicon oxide and 60 to 95 weight % aluminium oxide. Preferably, the carrier comprises or consists of 10 to 30 weight %, more preferably 15 to 25 weight % silicon oxide and 70 to 90 weight %, more preferably 75 to 85 weight % aluminium oxide. The carrier may comprise minor amounts of impurities or additional ingredients. In the carrier of the invention, the silicon oxide is SiO₂ and the aluminium oxide is Al₂O₃**.** In a preferred embodiment, the carrier consists of aluminium oxide and silicon oxide. In this respect, "consists" means that the material may comprise impurities, for instance up to 0.1, 0.5 or 2 weight %.

According to the invention, the coating does not comprise antimony. Of course, it cannot be excluded that low amounts of antimony are present due to impurities. However, antimony compounds are not actively added in the production process. Thus, the coating of the inventive catalyst may consist only of vanadium oxide and titanium oxide, optionally with additional molybdenum oxide, and may thus be produced from a low number of components. This facilitates the production process and decreases the risk of variations in composition and properties due to variations of the components included.

In a preferred embodiment of the invention, the catalyst does not comprise phosphorous. The catalyst can be prepared without adding a phosphorous salt, such as phosphorous acid and phosphates. In general, according to the invention, it is not necessary to include sources of inorganic materials, such as phosphorous, nitrogen or carbon sources. According to the invention, it is preferred that the coating essentially consists of metal oxides.

In another preferred embodiment, the catalyst does not comprise an alkali metal oxide. However, alkali metal oxides may be present due to impurities in known ratios, for instance up to 0.05, up to 0.1 or up to 0.5 weight %.

It is further preferred that no zirconium oxide is included. However, minor amounts of zirconium up to 0.05, 0.1 or 0.5 weight % may be present due to impurities. In another preferred embodiment of the invention, the catalyst does not comprise chromium oxide. In the production, no chromium source is added.

In further preferred embodiments, in the production process, no source of wolfram, iron, ruthenium, cobalt, rhodium, iridium, palladium, platinum, zinc and/or niobium is added. However, any of these metals may be present due to impurities in an amount of up to 0.05, 0.1 or 0.5 weight %. Preferably, no precious metals, such as palladium or platinum, are included.

Further, the inventive catalyst is preferably obtained without including a silicate, such as montmorillonite in the production process.

The carrier is provided in the form of particles, such as a granulate. The average diameter of the granules may be between 0.05 and 10 mm, preferably between 0.1 and 5 mm or between 0.5 and 2 mm. In a preferred embodiment, short strands of the carrier are used, for instance the strands may have a diameter between 0.2 and 3 mm, or between 0.5 and 1.5 mm, and a length of 2 to 10 mm, preferably 4 to 8 mm. In a preferred embodiment, the BET surface of the catalyst is between 5 to 25, more preferably between 8 to 15 m²/g. The BET surface is measured by the gas adsorption method according to DIN ISO 9277.

Carriers consisting of alumina and silica are available by Saint-Gobain under the trademark NorPro SA. A useful carrier consisting of 80 weight % Al₂O₃ and 20 weight % SiO₂ is NorPro SA 3235.
In a preferred embodiment, the bulk density of the catalyst is between 1740 and 1780 kg/m³ as measured by DIN ISO 697.

It is preferred that the carrier and the catalyst are porous. The contact area with the substrate can be increased when a porous catalyst is used in the inventive conversion of alkylpyridines to cyanopyridines. Preferably, the pore volume of the coated catalyst is between 0.2 to 1 cc/g, preferably between 0.3 to 0.6 cc/g, more preferably between 0.4 to 0.5 cc/g, as determined by Mercury intrusion porosimetry.

Another subject of the invention is a process for the production of a catalyst for the production of cyanopyridines from alkylpyridines, wherein the catalyst coating does not comprise antimony oxide, the process comprising in the order (a) to (d) the steps of
(a) providing a carrier,
(b) impregnating the carrier with an aqueous impregnating solution comprising a vanadium source and a titanium source,
(c) drying the catalyst,
(d) calcinating the catalyst.

Preferably, the inventive catalyst as disclosed above is prepared by the inventive process and the catalyst components are preferably selected as outlined above.

In a preferred embodiment of the invention, the aqueous impregnating solution in step (b) is obtained by
(i) providing a first aqueous solution comprising a vanadium source,
(ii) adding a weak organic acid to the first aqueous solution and mixing the solution,
(iii) providing a second aqueous solution comprising a titanium source,
(iv) mixing the first solution obtained in step (ii) with the second solution,
(v) heating at a temperature in the range of 40°C to 90°C.

According to the invention, a "vanadium source" or a "titanium source" is a vanadium compound or titanium compound, which can be coated on a carrier from aqueous solution and yields a vanadium oxide and titanium oxide coating after calcinating. Such materials are known in the art. The sources may be salts, complexes or organometallic precursors comprising the respective metal. Preferably, in the source, the metal has the same oxidative state as required for the catalyst, i.e. vanadium (5) or titanium (4).

In a preferred embodiment of the invention, the vanadium source comprises vanadium pentoxide or vanadium oxalate or ammonium metavanadate.

In step (b), an impregnation solution is applied to the carrier. As used herein, "solution" refers to a water-based solution or fine dispersion. Due to the high oxidative state of vanadium and titanium, vanadium and titanium compounds are not easily soluble. However, they are soluble or dispersible in aqueous media as specific complexes and under certain conditions. Although, the impregnation solution may be a finely distributed precipitate of the vanadium source or titanium source, a solution is preferred.

In a preferred embodiment of the invention, the weak organic acid used in step (ii) is oxalic acid. Preferably, a slurry of V₂O₅ in water is prepared under heating, for instance to 40 to 80°C under stirring. Oxalic acid is added slowly in order to obtain a vanadyl oxalate solution. The solution can be used as part of the impregnating solution in step (b).

In a preferred embodiment of the invention, the titanium source is titanium oxychloride. A 35-36 weight% titanium oxychloride solution in water and hydrochloric acid is commercially available from Millennium Inorganic Chemicals.

The addition of the weak organic acid in step (ii) is preferably carried out under temperature control, whereby the temperature is kept preferably between 30 and 90°C or 40 to 80°C, or at about 60°C. When adding the weak organic acid, an exothermic reaction occurs and the temperature has to be maintained in the desired range. The temperature is maintained for about 0.5 to 10 hours, preferably from 1 to 5 hours under stirring. Preferably, the temperature should not fall below 50°C in order to avoid precipitation.

When preparing the impregnating solution, the amount of titanium and vanadium is adjusted in view of the desired ratio in the catalyst. In the mixing step (iv), it is preferred to maintain the temperature at least at 50°C in order to avoid precipitation. Further, in order to avoid precipitation, the preparation of the impregnating solution (b) should be carried out with fresh solutions. Especially, in the first aqueous solution, the vanadium source should be used immediately after preparation.

In step (b), the carrier is impregnated with the aqueous impregnating solution. The amount of carrier and the amount of impregnating solution is adjusted such that the desired ratio of carrier to coating will be obtained in the catalyst. In a preferred embodiment, the impregnation step is carried out in a mixing device, such as a rotary coating drum. The temperature may be kept at room temperature.

In a preferred embodiment, the carrier is included into the rotary coating drum and the impregnating solution is poured into the drum subsequently. It is preferred to add the impregnating solution slowly, for instance in several steps, in order to allow the impregnating solution to diffuse into pores of the support. Preferably, the impregnating solution is added directly onto the carrier in the drum, such that sticking of the impregnating solution to the drum walls is avoided. In summary, care has to be taken that the carrier is intimately and evenly mixed with the coating solution and that it has sufficient time to take up the coating solution.

In a preferred embodiment, before adding the impregnating solution, the support (carrier) is slightly heated, for instance with a hot air blower. Preferably, the support is heated to about 30 to 50°C. When using lower temperatures, such as room temperature, precipitation may be observed during the addition of the impregnating solution, whereas at higher temperatures above 50°C, or even above 40°C, the carrier particles may stick to each other.

After the addition of all the impregnating solutions, the components are mixed further, for instance for at least 10 minutes or 30 minutes up to 2 or 3 hours. After a certain time, for instance about 1 hour, the material appears to be dry. It is then preferred to start heating the material, preferably with a hot air blower. The heating is continued under increasing temperature. Preferably, the final temperature is between 80 and 120°C, preferably about 90°C. At temperatures above 90°C, undesired dusting of the components may be observed. When increasing the temperature, care should be taken to avoid sticking of the components. It was found that sticking can be avoided when increasing the temperature at a rate of 0.2 to 0.8°C/min, preferably 0.4 to 0.5°C/min. Preferably, this rate is applied in the range of 30 to 80°C, or 40 to 60°C. The undesired sticking phenomenon can thus be avoided, which can occur when a decomposition, or strong evaporation, followed by a rapid solidification of the ingredients, occurs.

In a preferred embodiment of the invention, the drying step (c) is carried out under air and/or at a temperature between 25 and 100 °C, preferably 30 to 90°C, preferably under an increasing temperature rate.

In a preferred embodiment of the invention, the calcinating step (d) is carried out under air and/or at a temperature between 400-600°C, preferably 450 to 520°C, more preferably 460 to 480°C. The calcinating step (d) is preferably carried out for at least 4, at least 6 or at least 8 hours, preferably 4 up to 12 or up to 24 hours. The calcinating step can be carried out under air atmosphere. Preferably, an oven and devices are used which are resistant to HCI and Cl₂ vapours, such as titanium trays.

In another preferred embodiment of the invention, after the calcinating step (d), the material is sieved in a step (e). In this step, dust or lumps of support possibly formed during the production process are eliminated, and a homogeneous material is obtained. A sieving step may also be carried out before the drying step (c) and/or before the calcinating step (d).
A subject of the invention is a catalyst for the production of cyanopyridines from alkylpyridines, which is obtainable by the inventive process as disclosed above. Another subject of the invention is the use of the inventive catalyst for the production of cyanopyridines from alkylpyridines.

Another subject of the invention is a process for the production of cyanopyridines from alkylpyridines, comprising a step of contacting the alkylpyridines with an inventive catalyst as disclosed above. In a preferred embodiment of the invention, the contacting of the alkylpyridines and the catalyst is carried out in the presence of ammonia and oxygen. The method is thus an oxidative ammonolysis (ammoxidation).

Preferably, oxygen is supplied to the process by air. Alternatively, a specific oxygen content may be adjusted with molecular oxygen.

Methods for producing cyanopyridines from alkylpyridines by oxidative ammonolysis in the presence of catalysts are known in the art. Such processes, but with catalysts different from those used in the present invention, are disclosed for instance in WO 03/022819, WO 2005/016505, WO 2004/071657 or EP 0726092 A1. The processes for the production of cyanopyridines from alkylpyridines disclosed therein are incorporated by reference. Of course, in the process of the present invention, only the inventive catalyst is used.

In a preferred embodiment of the invention, the catalyst is provided in the form of a catalyst bed and/or at a temperature in the range of 250 to 450°C, preferably 320 to 450 °C or preferably 300 to 390°C. In a preferred embodiment of the invention, the alkylpyridine is contacted with the catalyst in the gaseous phase.

In a preferred embodiment of the invention, the alkylpyridine is 3-methylpyridine and the cyanopyridine is 3-cyanopyridine. In further embodiments of the invention, the alkylpyridine is 1-methylpyridine and the cyanopyridine is 1-cyanopyridine, or the alkylpyridine is 2-methylpyridine and the cyanopyridine is 2-cyanopyridine. It is also possible to use a mixture of alkylpyridines as starting components. Further, alkylpyridines having two or more alkyl moieties may be used, for instance lutidine.

In another embodiment of the invention, the molar ratio of the alkylpyridine to air is in the range of 1:10 to 1:80, preferably 1:20 to 1:70, and most preferably 1:20 to 1:65.

In another embodiment of the invention, the molar ratio of the alkylpyridine to ammonia is in the range of 1:1.5 to 1:15, preferably 1:2 to 1:8, and most preferably 1:2 to 1:6. The ratio of picoline/ammonia/oxygen is preferably 1:1-5:1.5-15.

The methods and catalysts of the invention solve the above-mentioned problem. The catalyst can be produced in a relatively simple manner from a limited number of starting components. The catalyst catalyzes the conversion of alkylpyridines to cyanopyridines efficiently and selectively. The products are obtainable in a high yield and high specificity. Due to the efficiency of the catalyst, it may be used at low temperatures. The catalyst is highly stable and thus can be used for an extended time period. Specifically, the catalyst has a good resistance to heat and reduction.

### Examples

### 1. Preparation of a catalysts

### 1.1 Vanadyl oxalate solution preparation

121 kg H₂O and 86.4 kg V₂O₅ are charged to a jacketed enamelled reactor and the slurry is heated up to 60°C under stirring. 189 kg oxalic acid are added very slowly during a time period of two to three hours in order to control the exothermic redox reaction and to keep the temperature at about 60°C. The temperature is maintained for another three hours under stirring to complete the reaction. Then, the solution is stored under stirring in the same reactor, whilst maintaining the temperature at 50°C in order to avoid precipitation. The amount of the solution thus obtained for each impregnation batch is that corresponding to 3.72 kg V₂O₅ per 30 kg of support, calculated from the vanadium content of the solution.

### 1.2 Titanium oxychloride solution

A solution (35 wt%) in water/hydrochloric acid is used as delivered by Millennium Pharmaceuticals ("titanium oxychloride - aqueous"). A predetermined quantity (1.49 kg per 30 kg of support) is used for every impregnation to get the desired final composition (see Ti/V/carrier ratio below).

### 1.3 Preparation of impregnation solution

The two solutions prepared as mentioned above are mixed under stirring just before starting the impregnation step to avoid any precipitation due to the instability of the mixed solution. In addition, the temperature is kept at 50°C to avoid precipitation.

### 2. Impregnation of the carrier and drying

As a carrier material, alumina/silica particles consisting of 80 wt% Al₂O₃ and 25 wt% SiO₂ (BET surface 12 m²/g) are used (trademark NorPro SA 3235, available from Saint Gobain). 30 kg of carrier (support) are loaded in a rotary coating drum maintained at room temperature. To avoid subsequent dusting, the carrier material is sieved previously. The support is slightly heated up to about 30 to 40°C with a hot air blower. The impregnating solution is quickly poured into the rotary coating drum preferably directly on the support. The procedure is performed in three or more steps. After each step, the addition of impregnating solution is interrupted, whilst mixing in order to allow diffusion of the highly vicious impregnating solution into the pores of the support. Thereby it is checked whether any sticking of the particles on the walls of the rotary drum occurs. In case sticking is observed, the material is left drying for a certain time before further impregnating solution is added.

Once the impregnating solution is fully poured into the rotary drum and the material appears to be dry (after about 1 hour), heating is started by means of a hot air blower. The operation is continued until the final temperature of at least 90°C is reached. The temperature increasing rate is set to 0.4 to 0.5°C in the range of 40 to 60°C. When adjusting the temperature increase accordingly, the coating is evenly attached on the carrier surface and a dried material is obtained.

### 3. Calcination

The dried material is unloaded from the rotary coating drum and immediately transferred into titanium trays suitable for calcination in an oven resistant to HCl and Cl₂ vapour. Each oven is suitable to collect, in three different trays, all the amount of the impregnated material coming from every single rotary coating drum. The oven temperature is directly set at the maximum temperature of 460 to 480°C. The material is calcinated under air atmosphere for at least 8 hours.

### 4. Sieving/Mixing

To eliminate dust and lumps of support formed during the production, the material is sieved. In order to have a more homogenous material, each single batch coming from each calcination oven is mixed.

### 5. Catalyst properties

The catalyst prepared according to the process above has the following properties:

| | |
|---|---|
| Bulk Density (finished catalyst) | 760 ± 20 kg/m³ |
| Friability Resistance (Roche attrition test) | ≥ 95% |
| Vanadium Oxide (SELLER's XRF analytical method GM035 or ICP-OES analytical method ZX-IP0235) | ≥ 8% V₂O₅ |
| Titanium Oxide (SELLER's XRF analytical method GM035 or ICP-OES analytical method ZX-IP0233) | ≥ 1% TiO₂ |
| Total active components | ≥ 10% V₂O₅ + TiO₂ |
| BET (nitrogen absorption, finished catalyst) | 10 - 17 m²/g |
| Pore volume (Mercury Intrusion Porosimetry, finished catalyst) | 0.4 - 0.5 cc/g |

For example, vanadium oxide contents of up to 15 % weight and titanium oxide contents up to 5 weight % may be obtained.

### 6. Production of 3-cyanopyridine from 3-methylpyridine

The catalyst was used in the conversion of 3-methylpyridine to 3-cyanopyridine. The 3-methylpyridine was vaporized at 150 - 250°C in a preheater and the vapours were allowed to pass over a catalyst bed maintained at a temperature in the range of 320-450°C. Ammonia and air are allowed to enter and mix with the educt above the catalyst bed. The reaction is carried out in a stainless steel reactor with 3 m length and 25 mm ID mounted in a vertical zone tubular furnace. The catalyst was packed in the reactor. Above the catalyst bed, packing of inert material such as porcelain or glass beads were placed to ensure proper distribution of the reactants. The reactor tube is heated and the temperature is maintained as per the requirement. The feeder line is provided with attachments, for air, for ammonia and for the methylpyridine. The first zone of the reactor tube packed with inert material acts as a vaporizer and super heater. Condenser traps and heater scrubbers are attached to the bottom outlet of the reactor in order to quench the product as soon as they exit the catalyst bed. The temperature of these condensers is maintained below 10°C by the circulation of chilled brine. The ratio of 3-picoline:ammonia:oxygen was 1 : 1.5 : 2.2.

### 7. Results:

It was found that the yield of 3-cyanopyridine initially and at a low oxygen level of 1:1.7 was about 95%. Even after continuous use of the catalyst for 6 years, the yield was still about 90%. During this time, it was not necessary to interrupt the process.

## Claims

1. A catalyst for the production of cyanopyridines from alkylpyridines, the catalyst comprising a carrier and a coating, wherein the coating comprises more than 50 weight % vanadium oxide and more than 2 weight % titanium oxide, wherein the coating does not comprise antimony oxide.

2. The catalyst of claim 1, wherein the coating further comprises molybdenum oxide.

3. The catalyst of at least one of the preceding claims, comprising
(a) 70 to 96. weight % carrier
(b) 3 to 25 weight % vanadium oxide
(c) 0.2 to 10 weight % titanium oxide
(d) 0 to 5 weight % molybdenum oxide.

4. The catalyst of at least one of the preceding claims, wherein the carrier comprises aluminium oxide and/or silicon oxide.

5. The catalyst of at least one of the preceding claims, wherein the carrier comprises 5 to 40 weight % silicon oxide and 60 to 95 weight % aluminium oxide.

6. A process for the production of a catalyst of claim 1 for the production of cyanopyridines from alkylpyridines, wherein the catalyst coating does not comprise antimony oxide, the process comprising in the order (a) to (d) the steps of
(a) providing a carrier,
(b) impregnating the carrier with an aqueous impregnating solution comprising a vanadium source and a titanium source,
(c) drying the catalyst,
(d) calcinating the catalyst.

7. The process of claim 6, wherein the aqueous impregnating solution in step (b) is obtained by
(i) providing a first aqueous solution comprising a vanadium source,
(ii) adding a weak organic acid to the first aqueous solution and mixing the solution,
(iii) providing a second aqueous solution comprising a titanium source,
(iv) mixing the first solution obtained in step (ii) with the second solution,
(v) heating at a temperature in the range of 40 to 90 C.

8. The process of at least one of the preceding claims, wherein the vanadium source comprises vanadium pentoxide or ammonium metavanadate.

9. The process of at least one of the preceding claims, wherein the weak organic acid is oxalic acid and/or the titanium source is titanium oxychloride.

10. The process of at least one of the preceding claims, wherein the drying step (c) is carried out under air and/or at a temperature between 25 and 100 °C.

11. The process of at least one of the preceding claims, wherein the calcinating step (d) is carried out under air and/or at a temperature between 400-600 °C.

12. The use of a catalyst of at least one of claims 1 to 5 for the production of cyanopyridines from alkylpyridines.

13. A process for the production of cyanopyridines from alkylpyridines, comprising a step of contacting the alkylpyridines with a catalyst according to at least one of claims 1 to 5.

14. The process of claim 13, wherein the contacting of the alkylpyridines and the catalyst is carried out in the presence of ammonia and oxygen.

15. The process of at least one of claims 13 and 14, wherein said catalyst is provided in the form of a catalyst bed and/or at a temperature in the range of 320 to 450°C and wherein the alkylpyridine is contacted with the catalyst in the gaseous phase.

16. The process of at least one of the preceding claims, wherein the alkylpyridine is 3-methylpyridine and the cyanopyridine is
3-cyanopyridine.
